# EUROPEAN PATENT SPECIFICATION

(11) **EP 1 680 072 B1**
(45) Date of publication and mention of the grant of the patent: **14.09.2011**
(21) Application number: 04790874.4
(22) Date of filing: 22.10.2004
(51) Int. Cl.: A61K 8/18

(54) **IMPROVED DETERGENT COMPOSITION WITH BENEFIT AGENTS**
VERBESSERTE DETERGENS-ZUSAMMENSETZUNG MIT VORTEILHAFTEN MITTELN
COMPOSITION DETERGENTE AMELIOREE COMPRENANT DES AGENTS BENEFIQUES

(30) Priority: 06.11.2003 IN MU11662003
(43) Date of publication of application: 19.07.2006
(73) Proprietor: Unilever PLC, London EC4Y 0DY (GB); Unilever N.V., 3013 AL Rotterdam (NL)
(72) Inventor: KRISHNAN, V.; c/o Hindustan Lever Ltd, Whitefield P.O., Bangalore 560 066 (IN); SHAH, Pankaj C.; c/o Hindustan Lever Ltd, Andheri (East), Mumbai 400 099 (IN); SIVAKUMAR, A.; c/o Hindustan Lever Ltd, Andheri (East), Mumbai 400 099 (IN)
(74) Representative: Elliott, Peter William
(86) International application number: PCT/EP2004/012091
(87) International publication number: WO 2005/044212

(56) References cited:
- WO-A-01/62224
- DE-A- 19 832 425
- US-A- 4 252 796
- US-A- 5 714 447
- US-B1- 6 241 976

## Description

The present invention relates to a non liquid (e.g. solid or bar) rinse-off cleansing composition, especially surfactant based high pH compositions having at least one pH sensitive benefit agent dispersed in a hydrophobic phase as a solid and/or as an aqueous solution/dispersion.

The invention thus provides for effective incorporation of pH sensitive benefit agents in various high pH cosmetics and/or detergent formulations, especially in high pH formulations which degrade and/or affect the benefit characteristics of the benefit agent when in direct contact with such high alkaline pH of the base formulation. The invention also relates to cosmetic/detergent formulations comprising protective pH sensitive benefit agents incorporated, so as to favour wider effective use/application of the benefit agents even in environments which are not usually compatible with respect to the basic constitution and character of the benefit agent.

It is well known that the cosmetic/detergent base formulations can determine the extent of efficacy of the formulation in imparting desired consumer attributes, in terms of the product form and usage benefits.

WO 02/087516 A2 discloses an antiperspirant stick formulation in which a particulate antiperspirant salt is suspended in a water immiscible carrier oil of which the major fraction is a hydrocarbon oil, the carrier oil is being solidified by a mixture of C14-C22 linear aliphatic monohydric alcohols, of which mixtures the C14-C16 alcohols represent from 15-65% by wt. Importantly, this patent is directed to a selective antiperspirant formulation to address problems of antiperspirant stick unevenness usually found in alcohol structured antiperspirant sticks, by providing a wax based stick employing a selective hydrocarbon oil as the main carrier fluid.

US 2003/0152539 A1 discloses an antiperspirant composition which is formulated based on the surprising finding that antiperspirant compositions containing petrolatum, a material that was previously known for inhibiting product wash-off and antiperspirant efficacy, can be formulated to deliver consumer perceived improvement in product wash-off and antiperspirant efficacy, provided that the petrolatum is formulated at relatively low concentrations of from about 0.05 % to about 0.95 %.

US-B-6 241 976 and US-A 4252796 disclose water-in-oil emulsions comprising benefit agents.

The above go to show the need for improving consumer attributes in various product formulations, both in terms of product form, as well as the efficacy in use/application. It is, however, experienced that whilst benefit agents and their incorporation in cosmetic/detergent base formulations is useful to impart consumer perceivable end use benefits and add value to the product, it is important to ascertain the compatibility of the benefit agent desired to be incorporated in the base formulation, as otherwise the presence of the benefit agent can degrade the product during storage, and/or affect the usual properties of the benefit agent.

US 6,306,806 discloses aqueous liquid cleansing and moisturising compositions, wherein the problem of interaction of the benefit agent with the base formulation is taken care of in providing a dual chamber dispenser, wherein a surfactant/base containing stripe is provided in one chamber, and a water-in-oil emulsion containing benefit stripe is provided in the other chamber, whereby only during use two separate stripes of the liquids in the respective chambers come out. Thus, the beneficial use of the benefit agent is achieved without allowing any direct interaction of the surfactant base and the benefit agent until use, to achieve enhanced deposition of the benefit agent.

It would be further apparent from the above US'806 reference that to maintain desired efficacy in terms of the deposition of the benefit agent during use, it is essential to keep the base liquid formulation and the benefit agent in physically separated chambers. Otherwise, when the water-in-oil emulsion containing the benefit agent is directly dispersed in the water based basic formulation, due to interaction of the base with the water-in-oil incorporated active benefit agent, there is clear loss of efficacy in deposition of active. This goes to show that even providing the active in a water-in-oil system is not sufficient for achieving desired deposition in use, and direct contact of the active in the water-in-oil emulsion is essentially to be avoided.

The above problems of non-compatibility of benefit agents when in direct contact with cosmetic and detergent formulations would be further evident from the fact that very often various product forms, apart from loss in efficacy in deposition of the active benefit agent, also show problems of instability/degradation in the product appearance. This can not only lead to loss of consumer appeal of the product, but can even render the product form not suitable for use.

It is thus well known that one needs to carefully select the benefit agent in the basic cosmetic/detergent formulation, in which the same is required to be incorporated and also the manner in which the benefit agent is to be incorporated in the base formulation, keeping in view also the product form and use.

Thus antiperspirant compositions which are usually low pH /mild formulations and can involve direct incorporation of various forms of antiperspirant agents such as aluminum containing and/or zirconium containing salts, or materials such as aluminum halides, aluminum chlorohydrate, aluminum hydroxyhalides, zirconyl oxyhalides, zirconly hydroxyhalides and mixtures thereof, for controlling or inhibiting underarm perspiration, wetness and odor.

It is found that when such antiperspirant actives are directly incorporated in alkaline high pH base formulations such as is usually in case of detergent base formulations, the antiperspirant active usually react and lose their identity in the product form. In particular, it is found that direct incorporation of Aluminum Chlorohydrate (ACH) solution, a well known antiperspirant active, in high pH detergent formulations was not possible because the same immediately reacted with the base formulation, and in the process resulted in precipitation of ACH.

Therefore, whilst such antiperspirants have their benefit characteristics which could well advantageously be introduced into detergent formulations, due to problems of incompatibility of the antiperspirant active with the alkaline detergent base the use of antiperspirant actives has been limited to only leave-on compositions. This necessarily is a clear limitation in the advantageous use of antiperspirant actives as benefit agents in detergent formulations.

Similar problems of incompatibility of benefit agents with certain cosmetic/detergent formulations leading to degradation of product are also noticed in direct incorporation of niacinamide, a skin whitening active, in soaps again with a high pH formulation. It is found that niacinamide when incorporated in soap at levels beyond 0.1 % interacts with the soap base, resulting in unwanted coloration of the product. This is again a major problem, since while use of niacinamide in soap could add value to the product, the coloration resulting from incorporation of niacinamide makes the soap bar aesthetically unacceptable. Importantly, since for effective use of niacinamide in such formulations, the same needs to be incorporated at levels beyond 0.1 %, it's presence in soap formulations is avoided due to the above mentioned discoloration and degradation of the product.

Also the incorporation of benefit agents such as high levels of inorganic salts in high pH soap formulations is found to lead to problems of efflorescence. Therefore, whilst such inorganic salts and their incorporation are supposed to serve a beneficial purpose and add value to the soap product, such problems of efflorescence limit their use and application in soap based formulations.

Also, in some end use applications of such inorganic salts such as low water usage products, the salts are preferably required to be released into water from the soap bar at a later stage to produce the desired effect, and add value and purpose in the incorporation of such benefit agents in the soap product. Therefore, not only is there problem in direct incorporation of such salts in high pH soap formulations, moreover their purpose may require delayed release of the active during use, which adds to the complexities of incorporating such actives in the soap product.

It would be clearly apparent from the above that whilst actives and their incorporation in soap formulations are advantageous and required to add value to the product, it is difficult to formulate high pH soap with direct incorporation of various benefit agents/actives which are not compatible with the soap base formulation, and lead to either degradation of the product and/or loss of benefit actives/characteristics of the benefit agent, making their incorporation of no use.

It is thus the basic object of the present invention to be able to provide for incorporation of pH sensitive benefit agents which may not be compatible for direct incorporation in non-liquid high pH base formulations such as alkaline soap base formulations. On the one hand this helps avoid problems of incompatibility and product degradation in use of such benefit agents experienced in the art as discussed above, and on the other hand also helps ensure that the efficacy of the benefit agent in the product form is maintained during storage and in subsequent use/application.

Another object of the present invention is to be able to provide for a simple and selective protective carrier for incorporating pH sensitive benefit agents/actives in high pH non-liquid soap formulations. On the one hand this would avoid any unwanted product degradation by interaction of the benefit agent with the soap base formulation until use of the formulation, and on the other hand this would also help ensure that the efficacy of the benefit agent in the product form is maintained during storage and in subsequent use/application.

Yet another object of the present invention is to be able to provide for formulations such as non-liquid soap/detergent formulations having high pH sensitive benefit agents/actives, whereby the soap composition can not only incorporate such pH sensitive incompatible benefit agents/actives, but would also be adapted to release such benefit agent only when required during use/application of the soap formulation, thereby maintaining desired efficacy. Yet another object of the present invention is to be able to provide for high detergent active based rinse-off non-liquid cleaning compositions incorporating incompatible benefit actives which may be selected from antiperspirant agents, skin lightening agents, perfuming agents, water softening agents, easy rinse-off agents and malodor controlling benefit agents, which would enable storage-stable incorporation of such actives and maintain desired efficacy in subsequent use/application of the product.

Thus according to a first aspect of the present invention, there is provided a rinse-off non-liquid cleansing composition comprising a continuous alkaline phase of one or more surfactants and a dispersed phase of a water-in-oil emulsion system comprising one or more hydrophobic materials, wherein at least one high pH sensitive water-soluble/dispersible benefit agent is dispersed in the hydrophobic phase. This will have the effect of maintaining the same storage stable and provide desired benefit action during use.

In accordance with a preferred aspect, there is provided a rinse-off cleansing composition comprising a continuous alkaline phase of one or more surfactants and a dispersed phase comprising one or more hydrophobic materials, wherein at least one high pH sensitive water-soluble/dispersible benefit agent is dispersed in the hydrophobic phase as a solid and/or as an aqueous solution/dispersion so as to maintain the same storage stable and desired benefit action during use.

In the above disclosed water-in-oil emulsion of the invention the active typically comprises 1 % to 70 % by wt. including at least one high pH sensitive water soluble and/or dispersible benefit agent; the hydrophobic protective carrier typically comprises 20 % to 95 % by wt.; and the emulsifier/dispersant typically comprises in amount of 0.5 % to 5 % by wt. Importantly, if the benefit agent is water-soluble and incorporated in the selective water-in-oil emulsion, then the actives can be added up to the solubility limit.

It is found that by way of the above invention that if the pH sensitive benefit agent is provided dispersed in the hydrophobic phase so as to maintain the same storage stability and desired benefit action during use, including the selective water-in-emulsion system, the same can be effectively stored free of any interaction, in combination with any high pH reactive base media, until the system is purposively subjected to any interactive actions, whereby the active is released from the system for the effective use/application.

The invention therefore not only favours the incorporation of the pH sensitive benefit agent in high pH formulations, but essentially serves the dual purpose of maintaining desired non-reactive storage of the high pH base and the pH sensitive benefit agent, as well as the desired efficacy during use, even in such adverse high pH environments.

In accordance with another aspect of the present invention, there is provided a rinse-off soap/detergent based cleansing composition comprising:
- 5 % to 80% detergent active comprising a continuous alkaline phase; and
- a dispersed phase comprising one or more hydrophobic materials, wherein at least one high pH sensitive water-soluble/dispersible benefit agent is dispersed in the hydrophobic phase so as to maintain the same storage stability and desired benefit action during use.

In accordance with another aspect of the present invention, there is provided a soap/detergent based composition comprising:
- 5 % to 80 % detergent active comprising a continuous alkaline phase;
- the dispersed phase comprising one or more hydrophobic materials including a water-in-oil emulsion system as a storage stable carrier of benefit agents/actives comprising (i) an aqueous phase including at least one water soluble and/or dispersible benefit agent, (ii) a hydrophobic protective carrier for the aqueous phase containing benefit agent/active, and (iii) an emulsifier/dispersant

In accordance with yet another preferred aspect of the present invention, there is provided a soap/detergent based composition comprising:
- 5 % to 80% detergent active comprising a continuous alkaline phase;
- the dispersed phase comprising one or more hydrophobic materials including a water-in-oil emulsion system as a storage stable carrier of benefit agents/actives comprising (i) an aqueous phase including at least one water soluble and/or dispersible benefit agent (ii) a hydrophobic protective carrier, preferably oil based, with or without oil soluble additives for the aqueous phase containing benefit agent/active, and (iii) an emulsifier/dispersant.

In the above dispersed phase comprising the benefit agent carrier of the invention, the benefit agent can range from any conventional available benefit agents such as antiperspirants, skin whitening agents, perfuming agents, water softening agents, rinse-off agents and malodor controlling benefit agents and other conventional benefit actives.

In accordance with some preferred aspects of the invention, the benefit agent can be selectively high pH sensitive ACH as an antiperspirant which, when incorporated in the dispersed phase comprising one or more hydrophobic materials and incorporated in a soap formulation, would avoid the problems of ACH precipitation usually encountered upon direct incorporation of ACH in alkaline soap formulations. Thus, the formulation of the invention would serve as a protective carrier for the active in the soap base involving incorporation of such antiperspirant in soap formulations to add consumer attributes to the soap product.

Likewise, using the above dispersed phase comprising one or more hydrophobic materials, it is possible to incorporate other usually non-compatible pH sensitive benefit agents in soap formulations such as Niacinamide (a skin whitening aid), phosphate ion (a water softening benefit agent) water-soluble inorganic salts such as Ca and Mg (as an easy to rinse benefit aid) and protein based actives (such as e.g. malodor preventing agents).

Depending upon the purpose and the selected base formulation used, the active is selectively provided in amounts such as to be maintained in storage stable state solubilized and/or dispersed in the oil phase.

In accordance with another aspect of the present invention, there is provided a process for manufacture of a rinse-off non-liquid cleansing composition comprising:
i) providing a continuous alkaline phase of one or more surfactants; and
ii) a dispersed phase comprising one or more hydrophobic materials, wherein at least one high pH sensitive water- soluble/dispersible benefit agent is dispersed in the hydrophobic phase so as to maintain the same storage stability and desired benefit efficacy during use.

The above process of manufacture involving the continuous alkaline phase and the dispersed phase including the benefit agent can be obtained following conventional non-liquid soap/detergent manufacture processes such as cast route, plodding and the like.

In accordance with another aspect of the present invention there is provided a process for manufacture of the a dispersed phase comprising:
i) providing a selective hydrophobic carrier, preferably oil based in the liquid state (under the conditions of emulsification/dispersion) and an emulsifier/dispersant, and mixing the same in the temperature range of 20-95°C;
ii) adding the benefit agent/active ingredient as a solution and/or a dispersion in oil phase;
iii) mixing the hydrophobic carrier and emulsifier mix with aqueous phase or powder to form a protective carrier for the benefit agent/active; and optionally providing the above carrier mix of (iii) in a base formulation.

In accordance with further aspect of the present invention, there is provided a process for the manufacture of non-liquid soap/detergent composition comprising:
i) providing the selective hydrophobic carrier, preferably oil based in the liquid state (under conditions of emulsification) with or without additional oil soluble actives and the emulsifier, and mixing the same in the temperature range of 20-95°C;
ii) adding the pH sensitive benefit agent/active ingredient as a solution and/or a dispersion in oil;
iii) mixing the hydrophobic carrier and emulsifier mix with the aqueous phase or powder to form the protective carrier for the benefit agent/active; and
iv) mixing the above protective carrier with the alkaline base detergent formulation.

The present invention thus provides for a rinse-off non-liquid cleansing composition comprising a continuous alkaline phase of one or more surfactants, and a dispersed phase comprising one or more hydrophobic materials, wherein at least one high pH sensitive water-soluble/dispersible benefit agent is dispersed in the hydrophobic phase so as to maintain the same storage stability and desired benefit efficacy during use.

It is found by way of the present invention that the problems of compatibility of some benefit agents, especially with the alkaline soap based formulations experienced in the art leading to instability of the product form in direct incorporation of pH sensitive actives, can be avoided if the benefit agent is provided in the protective carrier system such as being dispersed in a selective hydrophobic phase so as to maintain the same storage stability and desired benefit efficacy during use.

Importantly, the dispersed phase having the benefit agent can be a water-in-oil emulsion system in accordance with the present invention such that the active benefit agent containing aqueous solution is maintained emulsified in oil, or the active is dispersed in the oil. Thus, on the one hand the interaction of the active with the surroundings/soap base during storage is minimized, as there is an oil barrier between the active and the base formulation, and on the other hand desired efficacy of the benefit agent during use is maintained. It is only when in use that the oil barrier is selectively disturbed such that the active benefit agent is supposed to leach out of its protective form, and serve the purpose of the benefit agent in the formulation.

Advantageously, in the formulation of the invention the amount of the active in the dispersed phase having the benefit agent should be selectively provided, based on the selected benefit agent and the required end use/application. Importantly, in situations where the benefit agent is required to be released at a later point of time, the dispersed phase is such that it would facilitate maintaining the active in it's protected form within the hydrophobic carrier until it is desired to be exposed for the benefit application.

The basic system in terms of the aqueous composition, emulsifier level and oil concentration will typically be same for all variants of benefit agents desired to be included in the system. However, the amount of the active i.e. the benefit agent in the aqueous phase of the water-in-oil emulsion can be varied depending upon the extent of solubility or effective dispersibility of the active.

The soap/detergent base formulation can be any conventional soap/detergent formulation.

The dispersed phase would therefore essentially involve the hydrophobic carrier, which can be preferably selected from variety of oils including mineral oils, vegetable oils, silicone oils and synthetic oils.

The oil/hydrophobic carrier may be volatile or non-volatile.

A volatile oil may comprise a volatile hydrocarbon oil which evaporates during the process of drying skin or hair, and thereby releases the internal aqueous phase, which includes the first topically active compound to contact the skin or hair.

In one embodiment, the oil may comprise a combination of a volatile oil and a non-volatile oil. In this embodiment, an oil can be selectively provided to evaporate at a pre-selected temperature, and provide a controlled release of the first topically active compound at the pre-selected temperature. Pre-selected temperatures are those encountered during normal hair drying, provided for example by a hair dryer, or provided by a curling iron.

As previously stated, the oil also can include a water insoluble topically active compound in a sufficient amount to impart a particular functional or esthetic effect (e.g., emolliency), as long as the topically active compound does not adversely affect the emulsion composition (e.g. does not impart emulsion instability).

As previously stated, the oil also can be a non-volatile oil. The non-volatile oil can comprise a non-volatile silicone compound, a non-volatile hydrocarbon, or a mixture thereof. Preferably, the non-volatile oil comprises compounds which contain less than 50 % unsaturation. The nonvolatile oil does not evaporate from the skin or hair. The first topically active compound therefore is released by rubbing the skin or hair to rupture the primary water-in-oil emulsion. A non-volatile oil phase has a boiling point at atmospheric pressure of greater than about 250°C.

Exemplary non-volatile silicone compounds include a polyalkyl siloxane, a polyaryl siloxane or a polyalkylaryl siloxane. Mixes of these non-volatile silicone compounds also are useful.

The non-volatile oil also can comprise a non-volatile hydrocarbon compound, such as mineral oil, petrolatum, sunflower seed oil, canola oil or mixtures thereof. Other exemplary non-volatile hydrocarbon compounds that can be incorporated into the oil phase include, but are not limited to, a branched 1-decene oligomer, like 1-decene dimer or a polydecene.

The oil can also optionally comprise (1) an oil, such as jojoba oil, wheat germ oil or purcellin oil; or (2) a water insoluble emollient, such as, for example, an ester having at least about 10 carbon atoms, and preferably about 10 to about 32 carbon atoms.

Suitable esters include those comprising an aliphatic alcohol having about eight to about twenty carbon atoms, and an aliphatic or aromatic carboxylic acid including from two to about twelve carbon atoms, or conversely, an aliphatic alcohol having two to about twelve carbon atoms with an aliphatic or aromatic carboxylic acid including about eight to about twenty carbon atoms. The ester may be either straight chained or branched. Preferably, the ester has a molecular weight of less than about 500. Suitable esters therefore include, for example, but are not limited to:
(a) aliphatic monohydric alcohol esters (e.g., isopropyl isostearate, cetyl acetate, cetyl stearate); myristyl propionate, isopropyl myristate, isopropyl palmitate, cetyl acetate, cetyl propionate, cetyl stearate;
(b) aliphatic di- and tri-esters of polycarboxylic acids, (e.g. diisopropyl adipate);
(c) aliphatic polyhydric alcohol esters (e.g. propylene glycol dipelargonate); and
(d) aliphatic esters of aromatic acids, (e.g., C₁₂ -C₁₅ alcohol esters of benzoic acid).

Most preferably petrolatum can serve as an effective hydrophobic carrier in the emulsion system.

The emulsifier used in the water-in-oil emulsion can preferably be selected from low HLB emulsifier that may comprise a silicon-free surfactant, or a blend of silicon-free surfactants having an HLB value of about 10 or less (i.e. an HLB value of about 0.1 to about 10), an oil-soluble silicon-based surfactant, an oil-soluble polymeric surfactant, or mixtures thereof. Preferably, the silicon-free surfactant or surfactant blend has an HLB value of about 1 to about 7. To achieve the full advantage of the present invention, the silicon-free surfactant or surfactant blend preferably has an HLB value of about 3 to about 6. The term "oil-soluble" as used herein means a compound having a solubility of at least 0.1 g per 100 ml of oil phase to form a true solution.

The particularly preferred emulsifiers used in the water-in-oil emulsion are selected from polysorbate esters (e.g. sorbitan monooleate, sorbitan monostearate etc.), soya sterol, ethoxylated soya sterol, triglycerol diisostearate, oleic acid monoglyceride, mixture of high molecular weight fatty acids and fatty acid salts and mixed esters consisting of pentaerythritol and fatty alcohol.

The actives benefit agents as mentioned above can be any conventional benefit agents presently available in the art. This can be selected from antiperspirants such as ACH, niacinamide (a skin lightening aid), water soluble inorganic salts (easy rinse-off, water-softening benefit etc.) and protein based actives (as malodor preventing agent). The amount of actives can range up to 70 % of the system and in case of emulsion system, the water soluble actives in the aqueous phase are up to their solubility limits.

### EXAMPLES

The details of the invention its objects and advantages are explained hereunder in greater detail in relation to nonlimiting exemplary illustrations hereunder:

### Example 1

To demonstrate the pH dependant instability and precipitation of the antiperspirant active ACH in alkaline media which is usually the case when ACH is directly introduced in soap bases, the following experimentation was carried out.

A 1 % solution of ACH having initial pH of 4.6 was subjected to varying alkalinity by using sodium hydroxide solution, and the turbidity of the solution was measured at varying pH levels using a nephelometric turbidimeter. The results were noted for the varying pH level of the solution as detailed hereunder in Table I:

**TABLE I**

| pH | Turbidity, NTU |
|---|---|
| 4.6 | 1.3 |
| 5.7 | 2.6 |
| 7.5 | 504 |
| 9.6 | 522 |
| 10.6 | 884 |

It would be clearly apparent from the above that the active ACH solution was found to be unstable in alkaline conditions, and that ACH readily precipitates leading to higher turbidity at higher alkalinity. The above confirmed the reasons for preparation of ACH in soap base which are usually alkaline in nature.

### EXAMPLE 2

This illustration is directed to demonstrate the selective and advantageous application of the water-in-emulsion of the invention as a protective carrier for the ACH as an antiperspirant active in alkaline soap formulations, thereby facilitating the benefit use/applications of ACH in soaps. The following exemplary formulations were obtained:-

### Example 2a - Preparation of Soap Solution

A 1 % soap solution was prepared by dissolving the soap in distilled water at 70°C, and then cooling the solution to ambient temperature. The formulation of the soap used is given below:

| Soap Bar composition (% weight) | |
|---|---|
| Soap | 40 |
| Polyol | 30 |
| NSD | 7 |
| Perfume | 1 |
| Other | 4 |
| Water | 18 |

### Example 2b - Preparation of 100 mg ACH incorporated water-in-oil emulsion system in accordance with the invention

The dispersion or emulsification of ACH in petrolatum was prepared in the following way:
1. Dissolve ACH in distilled water to form a 60% solution.
2. Heat the petrolatum to 75°C and when melted, dissolve the emulsifier (sorbitan monooleate) in it at the desired concentration (2.5 %).
3. While stirring the petrolatum, the 60% ACH solution was added at the desired amount. In the case of a dispersion, ACH powder at the desired amount was slowly added.
4. After addition of the active, mixing was continued for 10 minutes, and then the emulsion/dispersion was cooled to ambient temperature whilst stirring.

To ascertain the stability of the ACH incorporated in the dispersed phase such as the water-in-oil emulsion in accordance with the invention prepared under Example 2b, the soap solution obtained under Example 2a was used, and the effect of direct addition of ACH and the ACH in the water-in-oil emulsion (Example 2b) of the invention was studied.

For the purpose of the above study, two separate batches of the same soap solution of Example 2a was taken. In one batch 60 mg ACH was directly added, and in the other batch 100 mg ACH was added through the water-in-oil emulsion system as per Example 2b above. The pH levels and the turbidity of the respective solutions containing the active ACH directly introduced (Control) and the active ACH in the water-in-oil emulsion (Example 2b) were measured, and the results are reproduced hereunder in Table II.

**TABLE II**

| Sample | pH | Turbidity |
|---|---|---|
| Soap solution | 10.14 | 205 |
| Soap + 60 mg ACH (Control) | 9.89 | 950 |
| Soap + 100 mg ACH through emulsion (Example 2b) | 10.13 | 185 |

It would be clearly apparent from the results in Table II above that when the ACH as the benefit agent is introduced in the soap solution through the water-in-oil emulsion of the invention, the problem of precipitation of ACH in the alkaline phase is clearly avoided, since the solution maintained a desired lower turbidity level.

On the other hand, as revealing from Table I and confirmed by Table II, the introduction of the active ACH directly into the soap solution resulted in precipitation of ACH·in the alkaline phase of the soap solution, and the high turbidity levels confirm the same. The above therefore clearly suggest the advantageous incorporation of active benefit agents in a protective form through a water-in-oil emulsion, which takes care of the required protection of the benefit agent in alkaline pH environments, so that effective use of such actives under varying pH conditions can be achieved.

Additional proof of the stability of ACH in our system is demonstrated in the example below.

### EXAMPLE 3

Under this example, 2.5 g of emulsion containing the ACH were immersed in 100 g of water or 100 gm of a 5 % soap solution. In one case, there was no mixing, and in the other case the water was stirred using a magnetic stirrer/overhead impeller. Water was drawn at desired time period, and analyzed for aluminum concentration using ICP. The results obtained are detailed hereunder in Table III

**TABLE III**

| Sample | Time, hours | ACH in water, % |
|---|---|---|
| Water - static | 2 | 0.45 |
| | 4 | 0.44 |
| | 6 | 0.45 |
| | 22 | 0.49 |
| | | |
| Water - mixing | 2 | 0.51 |
| | 4 | 0.53 |
| | 6 | 0.64 |
| | 22 | 0.65 |
| | | |
| Soap - mixing | 2 | 0.85 |
| | 4 | 0.88 |
| | 6 | 0.96 |
| | 22 | 1.19 |

The data clearly shows that even under agitation, the amount of ACH leached into water from the emulsion is less than 2 %.

Further studies were carried out to ascertain the efficacy of ACH incorporated in water-in-oil formulation carrier in soap formulation. For the purpose, a control soap base was used having the contents as in Table IV.

**Table IV**

| Control Soap Bar composition (% weight) | |
|---|---|
| Soap | 64 |
| Talc | 17 |
| NSD | 4 |
| Perfume | 1 |
| Other | 2 |
| Water | 12 |

In the same soap formulation of Table IV above ACH in water-in-oil emulsion was incorporated at a level of 4 %. The composition of the ACH in water-in-oil emulsion incorporated soap is detailed in Table V hereunder:

**TABLE V**

| ACH Soap Bar composition (%. weight) | |
|---|---|
| Soap | 59 |
| Talc | 14 |
| NSD | 4 |
| Perfume | 1 |
| ACH | 4 |
| Petrolatum | 2.5 |
| Sorbitan monooleate | 0.25 |
| Other | 2 |
| Water | 13.25 |

Both the control soap of Table IV and present soap having ACH in a water-in-oil emulsion of Table V were applied in 9 volunteers and the malodor on application of the two varieties of soaps were noticed. The rating was based on a scale of 0-5 with 0 signifying no modular and 5 being the worst. The results are noted in Table VI hereunder:

**TABLE - VI**

| Volunteer | Control Soap | Present Soap | Observations |
|---|---|---|---|
| 1 | 4 | 2 | Dry feeling for additional 2-3 hours; 50% body odour (BO) reduction |
| 2 | 3.5 | 1 | Dry for about 5-6 hrs; 50-60% BO reduction |
| 3 | 4 | 1.5 | Dry for at least 6 hours; 30-50% reduction in BO for 6 hours. |
| 4 | 4 | 2 | Dry for 6 hours; 60% reduction in BO |
| 5 | 4 | 1 | Dry for 1 hour; 95% reduction in BO for about 10 hours |
| 6 | 4 | 2 | No perspiration and BO for 4-5 hours after bath |
| 7 | 5 | 3 | Dry for 3 hours; 70% reduction in BO over 3-4 hours |
| 8 | 4 | 2 | Dry for 3-4 hours; 80% BO reduction for 7 hours |
| 9 | 5 | 1 | Dry for 1.5 hours; 80% BO reduction for 4-5 hours. |

As would be apparent from the above results, the soap contains the ACH in emulsion form accordance with the invention showed superior antiperspirant characteristics.

### EXAMPLE 4

### Clinical Study

The study was conducted with 37 volunteers, which is number required to analyse the results statistically. The control soap formulation as described in Table IV and the experimental soap formulation as described in Table V were used in the study. The products were completely randomized and had the same shape and perfume. The study was conducted for two weeks and the odour assessment was started on the third day after use of the soap.

The volunteers used the soap in the natural context of bathing, and then came to the assessment centre where expert assessors, who are trained to rate the malodour on a scale of 0-5, with 0 being no malodour, rated their underarm odour at 2, 4 and 8 hours post bath. The results of the study are summarized below in Table VII.

**Table VII**

| Duration | Control Soap | Experimental Soap | Significance |
|---|---|---|---|
| 2 hour | 1.0 | 0.7 | **** |
| 4 hour | 1.8 | 1.5 | *** |
| 8 hour | 1.9 | 1.7 | **** |

| | | | |
|---|---|---|---|
| ( *** - 99% and **** - 99.9%) | | | |

It is apparent from the above results that the soap containing the ACH in emulsion form accordance with the invention showed significant reduction in generation malodour in their underarm.

### EXAMPLE 5

### Stability of ACH in a bar

Certain benefit agents are highly pH sensitive for e.g. ACH, an antiperspirant which when incorporated in a soap formulation would cause the precipitation of ACH if it was incorporated directly in the alkaline soap. According to the present invention it has been found that dispersion of the high pH sensitive benefit agents in a hydrophobic phase ensures that the benefit agent is stable on storage in the soap base.

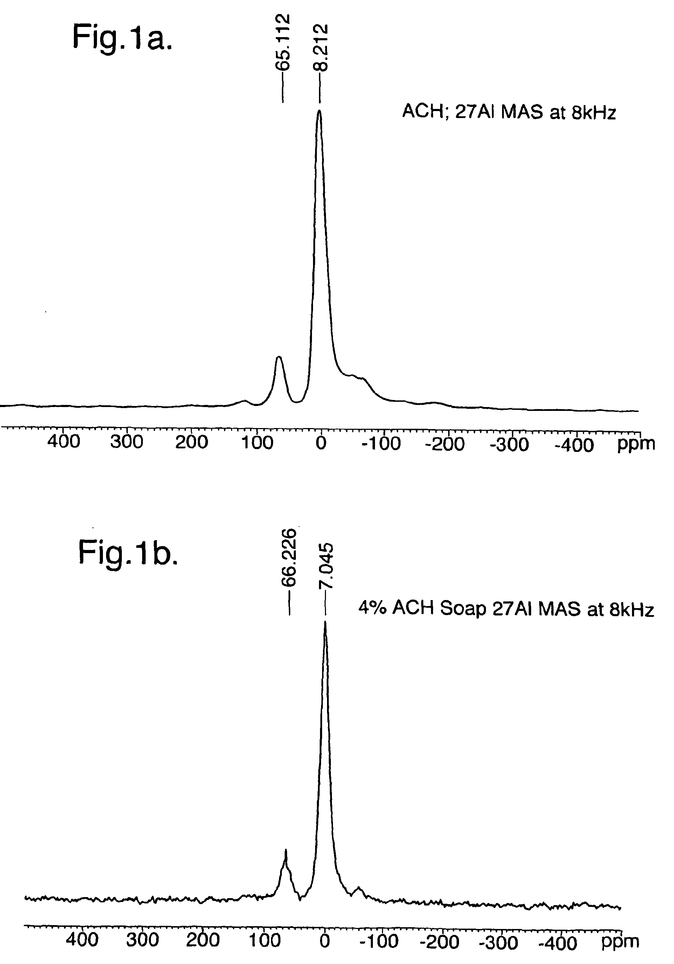

To demonstrate that the benefit agent for e.g. ACH was present unaffected in the alkaline environment of the soap, Solid State 27Al NMR was used to characterize the bars containing ACH. The spectra of the bar containing ACH in the emulsion (Figure 1b) is compared with that of neat ACH powder (Figure 1a). As seen from the spectra, the ACH remains as is in the bar. The same spectrum was obtained for bars that had been used by consumers for 3 weeks. Thus, the data clearly shows that even during use, as moisture seeps into the bar, there is no reaction of ACH with the soap and that the structured emulsion system protects it.

It is thus possible by way of the present invention to provide a rinse-off non liquid cleansing composition involving a continuous high pH alkaline phase and pH sensitive benefit agents as a dispersed phase comprising one or more hydrophobic materials. Importantly, the present invention is directed to the advantageous use of at least one high pH sensitive water-soluble/dispersible benefit agent dispersed in a selective hydrophobic carrier phase, which surprisingly not only maintain the same storage stable, but also of desired benefit efficacy during use. The invention would thus avoid the limitations of incorporating pH sensitive benefit agents in high pH alkaline soap/detergent formulations.

The invention would thus enable high pH alkaline soap base formulations to incorporate even pH sensitive benefit agents, avoiding problems of incompatibility and product degradation in the use of such benefit agents experienced in the art as discussed above. Importantly, also the soap composition according to the invention and the selective dispersed phase of the active/benefit agent would enable incorporation of even incompatible pH sensitive benefit agents/actives, which can be released only when required during use/application of the soap formulation, and maintained them storage stable when not in use.

## Claims

1. A rinse off non-liquid cleansing composition comprising A) a dispersed phase of a water-in-oil emulsion system as a storage stable carrier of high pH sensitivity benefit agents or actives comprising:
i) an aqueous phase including at least one water soluble and/or dispersible benefit agent;
ii) a hydrophobic protective carrier phase for the aqueous phase containing the benefit agent; and
iii) an emulsifier or a dispersant; and B) a continous alkaline phase

2. A composition according to claim 1 wherein the benefit agent is dispersed in the hydrophobic phase as a solid and/or as an aqueous solution/dispersion.

3. A composition according to claim 1 or claim 2 wherein the benefit agent is present in the range 1% to 70% by wt.

4. A composition according to any preceding claim wherein the hydrophobic protective carrier is present in the range 20% to 95% by wt.

5. A composition according to any preceding claim wherein the emulsifier/dispersant is present in the range 0.5% to 5% by wt.

6. A composition according to any preceding claim wherein the benefit agent is present up to the solubility limit of that agent.

7. A composition according to claim 6 wherein the benefit agent is water soluble.

8. A composition according to any preceding claim wherein the emulsifier is a polysorbate ester, soya sterol, ethoxylated soya sterol, triglycerol diisostearate, oleic acid glyceride, or a mixture of high molecular weight fatty acids and fatty acid salts and mixed esters comprising pentaerythritol and a fatty alcohol, or mixtures thereof.

9. A composition according to any preceding claim wherein the emulsifier has an HLB value of less than 10.

10. A composition according to any preceding claim wherein the emulsifier has an HLB value of 3 to 6.

11. A composition according to any preceding claim wherein the continuous alkaline phase comprises one or more surfactants.

12. A composition according to any preceding claim wherein the continuous alkaline phase comprises 5% to 80% detergent active and

13. A composition according to any preceding claim wherein the dispersed phase comprises a volatile and/or a non-volatile oil, which may be a mineral oil, a vegetable oil, a silicone oil or a synthetic oil, or mixtures thereof.

14. A composition according to any preceding claim wherein the dispersed phase comprises a non volatile hydrocarbon which is a branched 1-decene oligomer and may be selected from 1-decene dimer and polydecene, or a water soluble emollient which may be an ester having at least 8 carbon, atoms.

15. A composition according to claim 13 or claim 14 wherein the non-volatile oil comprises compounds which contain lass than 50% unsaturation.

16. A composition according to claims 13 to 15 wherein the non-volatile oil phase has a boiling point at atmospheric pressure of greater than 250°C.

17. A composition according to any preceding claim wherein the hydrophobic phase comprises petrolatum.

18. A compositions according to any preceding claim wherein the benefit agent is selected from antiperspirants, skin whitening agents, perfuming agents, water softening agents, rinse-off agents, malodour controlling benefit agents and protein based actives.

19. The composition of claim 18 wherein the benefit agent is a high pH sensitive Aluminium chlorohydrate antiperspirant salt, niacinamide, phosphate ion, or a calcium or magnesium salt.

20. The composition of claim 13 wherein the oil is selected to evaporate at a pre-selected temperature and provide controlled release/deposition of a topically active compound at a pre-selected temperature.

21. A process of providing an emulsion system of composition of any of the preceding claims, wherein the dispersed phase is prepared by the steps of;
i) providing of selective hydrophobic carrier preferably oil based in the liquid state (under the conditions of emulsification/dispersion) and an emulsifier/dispersant and mixing the same in the temperature range of 20-95°C;
ii) adding the benefit agent/active ingredient as a solution and/or a dispersion in oil phase;
iii) mixing the hydrophobic carrier and emulsifier mix with aqueous phase or powder to form the protective carrier for the benefit agent/active and optionally; and
iv) providing the above carrier mix of (iii) in a base formulation.

22. The process of claim 21 wherein additional oil soluble actives are present.

23. The process of claim 21 or claim 22 wherein the emulsion system is mixed into an alkaline base detergent formulation.

24. The process of claim 23 wherein the emulsion system is mixed into an alkaline base detergent formulation by either casting or plodding.

## Patentansprüche

1. Nicht-flüssige Reinigungszusammensetzung zum Abspülen, umfassend
A) eine dispergierte Phase aus einem Wasser-in-Öl-Emulsionssystem als lagerungsstabiler Träger von Wirkstoffen oder aktiven Mitteln mit Sensitivität gegenüber hohem pH, umfassend:
i) eine wässrige Phase, umfassend wenigstens einen in Wasser löslichen und/oder dispergierbaren Wirkstoff;
ii) eine hydrophobe protektive Trägerphase für die wässrige Phase, die den Wirkstoff enthält, und
iii) einen Emulgator oder ein Dispergiermittel; und
B) eine kontinuierliche alkalische Phase.

2. Zusammensetzung gemäß Anspruch 1, wobei der Wirkstoff in der hydrophoben Phase als Feststoff und/oder als wässrige Lösung / Dispersion dispergiert ist.

3. Zusammensetzung gemäß Anspruch 1 oder Anspruch 2, wobei der Wirkstoff im Bereich von 1 bis 70 Gewichts-% vorliegt.

4. Zusammensetzung gemäß einem vorangehenden Anspruch, wobei der hydrophobe protektive Träger im Bereich von 20 bis 95 Gewichts-% vorliegt.

5. Zusammensetzung gemäß einem vorangehenden Anspruch, wobei der Emulgator / das Dispergiermittel im Bereich von 0,5 bis 5 Gewichts-% vorliegt.

6. Zusammensetzung gemäß einem vorangehenden Anspruch, wobei der Wirkstoff bis zur Löslichkeitsgrenze dieses Mittels vorliegt.

7. Zusammensetzung gemäß Anspruch 6, wobei der Wirkstoff in Wasser löslich ist.

8. Zusammensetzung gemäß einem vorangehenden Anspruch, wobei der Emulgator ein Polysorbatester, Sojasterol, ethoxyliertes Sojasterol, Triglycerindiisostearat, Ölsäureglycerid oder ein Gemisch aus Fettsäuren mit hohem Molekulargewicht und Fettsäuresalzen und gemischten Estern, umfassend Pentaerythritol und einen Fettalkohol, oder Gemische davon ist.

9. Zusammensetzung gemäß einem vorangehenden Anspruch, wobei der Emulgator einen HLB-Wert von kleiner als 10 hat.

10. Zusammensetzung gemäß einem vorangehenden Anspruch, wobei der Emulgator einen HLB-Wert von 3 bis 6 hat.

11. Zusammensetzung gemäß einem vorangehenden Anspruch, wobei die kontinuierliche alkalische Phase ein oder mehrere Surfactant(s) umfasst.

12. Zusammensetzung gemäß einem vorangehenden Anspruch, wobei die kontinuierliche alkalische Phase 5 bis 80 % aktives Detergent umfasst.

13. Zusammensetzung gemäß einem vorangehenden Anspruch, wobei die dispergierte Phase ein flüchtiges und/oder ein nicht-flüchtiges Öl umfasst, das ein Mineralöl, ein Pflanzenöl, ein Silikonöl oder ein synthetisches Öl oder Gemische davon sein kann.

14. Zusammensetzung gemäß einem vorangehenden Anspruch, wobei die dispergierte Phase einen nicht-flüchtigen Kohlenwasserstoff, der ein verzweigtes 1-Decen-Oligomer ist und aus 1-Decen-Dimer und Polydecen ausgewählt sein kann, oder ein wasserlösliches Emolliens, das ein Ester mit wenigstens 8 Kohlenstoffatomen sein kann, umfasst.

15. Zusammensetzung gemäß Anspruch 13 oder Anspruch 14, wobei das nicht-flüchtige Öl Verbindungen umfasst, die weniger als 50 % Ungesättigtheit enthalten.

16. Zusammensetzung gemäß den Ansprüchen 13 bis 15, wobei die nicht-flüchtige Ölphase bei Atmosphärendruck einen Siedepunkt von höher als 250 °C hat.

17. Zusammensetzung gemäß einem vorangehenden Anspruch, wobei die hydrophobe Phase Petrolatum umfasst.

18. Zusammensetzung gemäß einem vorangehenden Anspruch, wobei der Wirkstoff aus Antiperspirantien, Hautbleichmitteln, parfümierenden Mitteln, Wasser weichmachenden Mitteln, Glanztrocknungshilfen, Wirkstoffen zur Bekämpfung von schlechtem Geruch und aktiven Mitteln auf Proteinbasis ausgewählt ist.

19. Zusammensetzung gemäß Anspruch 18, wobei der Wirkstoff ein gegenüber hohem pH empfindliches Aluminiumchlorhydrat-Antiperspirant-Salz, Niacinamid, Phosphation oder ein Calcium- oder Magnesiumsalz ist.

20. Zusammensetzung gemäß Anspruch 13, wobei das Öl so ausgewählt ist, dass es bei einer vorgewählten Temperatur verdampft und eine kontrollierte Freisetzung / Abscheidung einer topisch aktiven Verbindung bei einer vorgewählten Temperatur bereitgestellt wird.

21. Verfahren zum Bereitstellen eines Emulsionssystems einer Zusammensetzung gemäß einem der vorangehenden Ansprüche, wobei die dispergierte Phase hergestellt wird durch die Schritte:
i) Bereitstellen eines selektiven hydrophoben Trägers, vorzugsweise auf Ölbasis, in flüssigem Zustand (unter den Bedingungen einer Emulgierung / Dispersion) und eines Emulgators / Dispergiermittels und Mischen derselben im Temperaturbereich von 20 bis 95 °C;
ii) Zusetzen des Wirkstoffs / aktiven Ingrediens als eine Lösung und/oder eine Dispersion in Ölphase;
iii) Mischen des hydrophoben Trägers und der Emulgatormischung mit wässriger Phase oder Pulver unter Bildung des protektiven Trägers für den Wirkstoff / das aktive Mittel und gegebenenfalls
iv) Bereitstellen der obigen Trägermischung von iii) in einer Basenformulierung.

22. Verfahren gemäß Anspruch 21, wobei zusätzliche öllösliche aktive Mittel vorhanden sind.

23. Verfahren gemäß Anspruch 21 oder Anspruch 22, wobei das Emulsionssystem in eine alkalische Basen-Detergent-Formulierung gemischt wird.

24. Verfahren gemäß Anspruch 23, wobei das Emulsionssystem durch Gießen oder Einarbeiten in die alkalische Basen-Detergent-Formulierung gemischt wird.

## Revendications

1. Composition de nettoyage non liquide à rincer comprenant
A) une phase dispersée d'un système d'émulsion eau dans l'huile à titre de vecteur stable au stockage d'agents bénéfiques ou de substances actives ayant une sensibilité élevée au pH comprenant :
i) une phase aqueuse contenant au moins un agent bénéfique soluble et/ou dispersible dans l'eau ;
ii) une phase d'un vecteur protecteur hydrophobe, pour la phase aqueuse contenant l'agent bénéfique ; et
iii) un émulsifiant ou un dispersant ; et
B) une phase alcaline continue.

2. Composition selon la revendication 1, dans laquelle l'agent bénéfique est dispersé dans la phase hydrophobe sous la forme d'un solide et/ou d'une solution/dispersion aqueuse.

3. Composition selon la revendication 1 ou la revendication 2, dans laquelle l'agent bénéfique est présent dans la plage de 1 à 70 % en poids.

4. Composition selon une revendication précédente quelconque, dans laquelle le vecteur protecteur hydrophobe est présent dans la plage de 20 à 95 % en poids.

5. Composition selon une revendication précédente quelconque, dans laquelle l'émulsifiant/dispersant est présent dans la plage de 0,5 à 5 % en poids.

6. Composition selon une revendication précédente quelconque, dans laquelle l'agent bénéfique est présent jusqu'à la limite de solubilité dudit agent.

7. Composition selon la revendication 6, dans laquelle l'agent bénéfique est soluble dans l'eau.

8. Composition selon une revendication précédente quelconque, dans laquelle l'émulsifiant est un ester de polysorbate, un stérol de soja, un stérol de soja éthoxylé, un diisostéarate de tri-glycérol, un glycéride d'acide oléique, ou un mélange d'acides gras et de sels d'acides gras de poids moléculaire élevé et des esters mélangés comprenant du pentaérythritol et un alcool gras ou des mélanges de ceux-ci.

9. Composition selon une revendication précédente quelconque, dans laquelle l'émulsifiant a un indice HLB inférieur à 10.

10. Composition selon une revendication précédente quelconque, dans laquelle l'émulsifiant a un indice HLB de 3 à 6.

11. Composition selon une revendication précédente quelconque, dans laquelle la phase alcaline continue comprend un ou plusieurs tensioactifs.

12. Composition selon une revendication précédente quelconque, dans laquelle la phase alcaline continue comprend de 5 à 80 % de substance active détergente.

13. Composition selon une revendication précédente quelconque, dans laquelle la phase dispersée comprend une huile volatile et/ou non volatile qui peut être une huile minérale, une huile végétale, une huile de silicone ou une huile synthétique, ou des mélanges de celles-ci.

14. Composition selon une revendication précédente quelconque, dans laquelle la phase dispersée comprend un hydrocarbure non volatil qui est un oligomère de 1-décène ramifié et peut être choisi parmi un dimère de 1-décène ou un polydécène, ou un émollient soluble dans l'eau qui peut être un ester ayant au moins 8 atomes de carbone.

15. Composition selon la revendication 13 ou la revendication 14, dans laquelle l'huile non volatile comprend des composés contenant moins de 50 % d'insaturation.

16. Composition selon les revendications 13 à 15, dans laquelle la phase huile non volatile a un point d'ébullition à la pression atmosphérique supérieur à 250°C.

17. Composition selon une revendication précédente quelconque, dans laquelle la phase hydrophobe comprend de la vaseline.

18. Composition selon une revendication précédente quelconque, dans laquelle l'agent bénéfique est choisi parmi les antiperspirants, les agents blanchissants de la peau, les agents parfumants, les agents d'adoucissement de l'eau, les agents de rinçage, les agents bénéfiques de lutte contre les mauvaises odeurs et les substances actives à base de protéines.

19. Composition selon la revendication 18, dans laquelle l'agent bénéfique est un sel antiperspirant de type chlorhydrate d'aluminium ayant une sensibilité élevée au pH, un niacinamide, un ion phosphate, ou un sel de calcium ou de magnésium.

20. Composition selon la revendication 13, dans laquelle l'huile est choisie pour s'évaporer à une température présélectionnée et permet la libération/le dépôt contrôlé(e) d'un composé à action topique à une température présélectionnée.

21. Procédé de préparation d'un système d'émulsion de la composition selon l'une quelconque des revendications précédentes, dans lequel la phase dispersée est préparée par les étapes de :
i) préparer un vecteur hydrophobe sélectif, de préférence, à base d'huile à l'état liquide (dans des conditions d'émulsification/dispersion) et un émulsifiant/dispersant et les mélanger dans la plage de températures de 20 à 95°C ;
ii) ajouter l'agent bénéfique/la substance active sous la forme d'une solution et/ou d'une dispersion dans la phase huile ;
iii) mélanger le vecteur hydrophobe et le mélange émulsifiant avec la phase aqueuse ou une poudre pour former le vecteur protecteur pour l'agent bénéfique/la substance active ; et éventuellement
iv) utiliser le mélange de vecteurs obtenu en (iii) ci-dessus dans une formulation de base.

22. Procédé selon la revendication 21, dans lequel des substances actives solubles dans l'huile additionnel sont présentes.

23. Procédé selon la revendication 21 ou la revendication 22, dans lequel le système d'émulsion est mélangé dans une formulation détergente de base de nature alcaline.

24. Procédé selon la revendication 23, dans lequel le système d'émulsion est mélangé dans une formulation détergente de base de nature alcaline soit par coulée, soit par passage dans une boudineuse.
